Europäisches Patentamt

European Patent Office

Office européen des brevets

(11) Publication number : **0 622 081 A2**

(12) # EUROPEAN PATENT APPLICATION

(21) Application number : 94302767.2

(22) Date of filing : 19.04.94

(51) Int. Cl.$^5$ : **A61K 39/09,** C07K 13/00, C12Q 1/68

(30) Priority : 20.04.93 US 48896

(43) Date of publication of application : 02.11.94 Bulletin 94/44

(84) Designated Contracting States : AT BE CH DE DK ES FR GB GR IE IT LI LU MC NL PT SE

(71) Applicant : UAB RESEARCH FOUNDATION P.O. Box 1000 Birmingham Alabama 35294 (US)

(72) Inventor : Briles, David E. 760 Linwood Road Birmingham, Alabama 35222 (US) Inventor : Yother, Janet L. 2208 Heatherbrooke Road Birmingham, Alabama 35242 (US) Inventor : McDaniel, Larry S. 5354 Cornell Drive Birmingham, Alabama 35210 (US)

(74) Representative : Smart, Peter John W.H. BECK, GREENER & CO 7 Stone Buildings Lincoln's Inn London WC2A 3SZ (GB)

(54) Epitopic regions of phneumococcal surface protein A.

(57) A region of the PspA protein of the Rxl strain of protection-eliciting eptiopes which are cross-reactive with PspAs of other S. pneumoniae strains. The region comprises the 68 amino acid sequence extending from amino acid residues 192 to 260 of the Rx1 PspA strain.

EP 0 622 081 A2

This invention relates to protein fragments containing epitopic regions of pneumococcal surface protein A (PspA), the major virulence factor of Streptococcus pneumoniae.

Streptococcus pneumoniae is an important cause of otitis media, meningitis, bacteremia and pneumonia. Despite the use of antibiotics and vaccines, the prevalence of pneumococcal infections has declined little over the last twenty-five years.

It is generally accepted that immunity to Streptococcus pneumoniae can be mediated by specific antibodies against the polysaccharide capsule of the pneumococcus. However, neonates and young children fail to make an immune response against polysaccharide antigens and can have repeated infections involving the same capsular serotype.

One approach to immunising infants against a number of encapsulated bacteria is to conjugate the capsular polysaccharide antigens to proteins to make them immunogenic. This approach has been successful, for example, with Haemophilus influenzae b (see U.S. Patent No. 4,496,538 to Gordon and U.S. Patent No. 4,673,574 to Anderson). However, there are over eighty known capsular serotypes of S. pneumoniae of which twenty-three account for most of the disease. For a pneumococcal polysaccharide-protein conjugate to be successful, the capsular types responsible for most pneumococcal infections would have to be made adequately immunogenic. This approach may be difficult, because the twenty-three polysaccharides included in the presently-available vaccine are not all adequately immunogenic, even in adults. Furthermore, such a vaccine would probably be much more expensive to produce than any of the other childhood vaccines in routine use.

An alternative approach for protecting children, and also the elderly, from pneumococcal infection would be to identify protein antigens that could elicit protective immune responses. Such proteins may serve as a vaccine by themselves, may be used in conjunction with successful polysaccharide-protein conjugates, or as carriers for polysaccharides.

In McDaniel et al (I), J. Exp. Med. 160:386-397, 1984, there is described the production of hybridoma antibodies that recognize cell surface proteins on S. pneumoniae and protection of mice from infection with certain strains of encapsulated pneumococci by such antibodies. This surface protein antigen has been termed "pneumococcal surface protein A" or PspA for short.

In McDaniel et al (II), Microbial Pathogenesis 1:519-531, 1986, there are described studies on the characterization of the PspA. From the results of McDaniel (II), McDaniel (III), J.Exp. Med. 165:381-394, 1987, Waltman et al., Microb. Pathog. 8:61-69, 1990 and Crain et al., Infect. Immun. 58: 3293-3299, 1990, it was also apparent that the PspAs of different strains frequently exhibit considerable diversity in terms of their epitopes, and apparent molecular weight.

In McDaniel et al (III), there is disclosed that immunization of X-linked immunodeficient (XID) mice with non-encapsulated pneumococci expressing PspA, but not isogenic pneumococci lacking PspA, protects mice from subsequent fatal infection with pneumococci.

In McDaniel et al (IV), Infect. Immun., 59:222-228, 1991, there is described immunization of mice with a recombinant full length fragment of PspA that is able to elicit protection against pneumococcal strains of capsular types 6A and 3.

In Crain et al, (supra) there is described a rabbit anti-serum that detects PspA in 100% (n = 95) of clinical and laboratory isolates of strains of S. pneumoniae. When reacted with seven monoclonal antibodies to PspA, fifty-seven S. pneumoniae isolates exhibited thirty-one different patterns of reactivity. Accordingly, although a large number of serologically-different PspAs exist, there are extensive cross-reactions between PspAs.

The PspA protein type is independent of capsular type. It would seem that genetic mutation or exchange in the environment has allowed for the development of a large pool of strains which are highly diverse with respect to capsule, PspA, and possibly other molecules with variable structures. Variability of PspA's from different strains also is evident in their molecular weights, which range from 67 to 99 kD. The observed differences are stably inherited and are not the result of protein degradation.

Immunization with a partially purified PspA from a recombinant λ2 gtII clone, elicited protection against challenge with several S. pneumoniae strains representing different capsular and PspA types, as described in McDaniel et al (IV), Infect. Immun. 59:222-228, 1991. Although clones expressing PspA were constructed according to that paper, the product was insoluble and isolation from cell fragments following lysis was not possible.

While the protein is variable in structure between different pneumococcal strains, numerous cross-reactions exist between all PspA's, suggesting that sufficient common epitopes may be present to allow a single PspA or at least a small number of PspA's to elicit protection against a large number of S. pneumoniae strains.

In addition to the published literature specifically referred to above, the inventors, in conjunction with co-workers, have published further details concerning PspA's, as follows:

1. Abstracts of 89th Annual Meeting of the American Society for Microbiology, p.125, item D-257, May 1989;

2. Abstracts of 90th Annual Meeting of the American Society for Microbiology, p.98, item D-106, May 1990;

3. Abstracts of 3rd International ASM Conference on Streptococcal Genetics, p.11, item 12, June 1990 ;

4. Talkington et al, Infect. Immun. 59:1285-1289, 1991;

5. Yother et al (I), J. Bacteriol. 174:601-609, 1992;

6. Yother et al (II), J. Bacteriol. 174:610-618, 1992; and

7. McDaniel et al (V), Microbiol Pathogenesis, 13:261-268.

In the co-pending United States patent applications Serial Nos. 656,773 and 835,698 (corresponding to published WO 92/14488) as well as in Yother et al (I) and (II), there are described the preparation of mutants of S. pneumoniae that secrete an immunogenic truncated form of the PspA protein, and the isolation and purification of the secreted protein. The truncated form of PspA was found to be immunoprotective and to contain the protective epitopes of PspA. The PspA protein described wherein is soluble in physiologic solution and lacks at least the functional cell membrane anchor region.

In the specification which follows and the drawings accompanying the same, there are utilized certain accepted abbreviations with respect to the amino acids represented thereby. The following Table I identifies whose abbreviations:

**TABLE I**

| AMINO ACID ABBREVIATIONS | |
| --- | --- |
| A = Ala = Alanine | M = Met = Methionine |
| C = Cys - Cysteine | N = Asn = Asparagine |
| D = Asp = Aspartic Acid | P = Pro = Proline |
| E = Glu = Glutamic Acid | Q = Gln = Glutamine |
| F = Phe = Phenylalanine | R = Arg = Arginine |
| G = Gly = Glycine | S = Ser = Serine |
| H = His = Histidine | T = Thr = Threonine |
| I = Ile = Isoleucine | V = Val = Valine |
| K = Lys = Lysine | W = Try = Tryptophen |
| L = Leu = Leucine | Y = Tyr = Tyrosine |

In accordance with the present invention, there has been identified a 68-amino acid region of PspA from the Rx1 strain of Streptococcus pneumoniae which not only contains protection-eliciting epitopes, but also is sufficiently cross-reactive with other PspA's from other S. pneumoniae strains so as to be a suitable candidate for the region of PspA to be incorporated into a recombinant PspA vaccine.

The 68-amino acid sequence extends from amino acid residues 192 to 260 of the Rx1 PspA protein. While the disclosure herein refers particularly to the specific 68 amino acid sequence of the Rx1 PspA protein, any region of a PspA protein from any other S. pneumoniae species which is homologous to this sequence of the Rx1 PspA protein is included within the scope of the invention, for example, from strains D39 and R36A.

Accordingly, in one aspect, the present invention provides an isolated pneumococcal surface protein A (PspA) protein fragment comprising amino acid residues corresponding to all or some of amino acid residues 192 to 260 of the PspA protein of the Rx1 strain of Streptococcus pneumoniae containing at least one protection-eliciting epitope and optionally up to a further 25 residues of said protein in the $NH_2$ terminal direction and/or the COOH terminal direction, or being effectively homologous with such a protein fragment.

The protein fragment may be one containing an amino acid sequence corresponding to or homologous to the amino acid residues 192 to 260 of the PspA protein of the Rx1 strain and hence may comprise fragments larger or smaller than ones containing the specific amino acid sequence.

The protein fragment of the invention may be produced recombinantly in the form of a truncated C-terminal deleted product containing the protein fragment, specifically a truncated C-terminal-deleted product containing the approximately C-terminal third of an α-helical region of the native PspA protein.

The amino acid sequence of the protein fragment need not be that found in strain Rx1 but can be based on a corresponding sequence from another strain. Thus, the present invention also includes an isolated protein fragment comprising an amino acid sequence corresponding to that of a protein-eliciting epitope contained in amino acid residues 192 to 260 of the PspA protein of the Rx1 strain of Streptococcus pneumoniae.

In particular, the invention includes an isolated protein fragment comprising the amino acid sequence of or effectively homologous with that of a protection-eliciting epitope corresponding to an epitope contained in amino acid residues 192 to 260 of the pneumococcal surface protein A (PspA) protein of the Rx1 strain of Streptococcus pneumoniae, and including no more than 25 additional amino acid residues in the $NH_2$ and/or the COOH terminal direction.

The invention includes a vaccine containing a protein fragment of the invention. It also includes certain DNA primers or probes described herein.

The invention will be further described with reference to the following drawings in which:-

Figure 1 contains the DNA sequence for the pspA gene of the Rx1 strain of S. pneumoniae with the deduced amino acid sequence for the PspA protein;

Figure 2 contains a schematic representation of the domains of mature PspA protein as well as identification of certain plasmids containing gene sequences coding for the full length protein (pKSD 1014), coding for specific segments of the N-terminal portion of the protein (pJY4284 or pJY4285, pJY4310, pJY4306) and coding for specific sequences of the C-terminal region of the protein (pBC207, pBC100);

Figure 3 contains a schematic representation of the domains of the mature PspA protein and the general location of epitopes recognised by certain monoclonal antibodies; and

Figure 4 is an immunoblot of PspA protein gene products produced by plasmids identified therein.

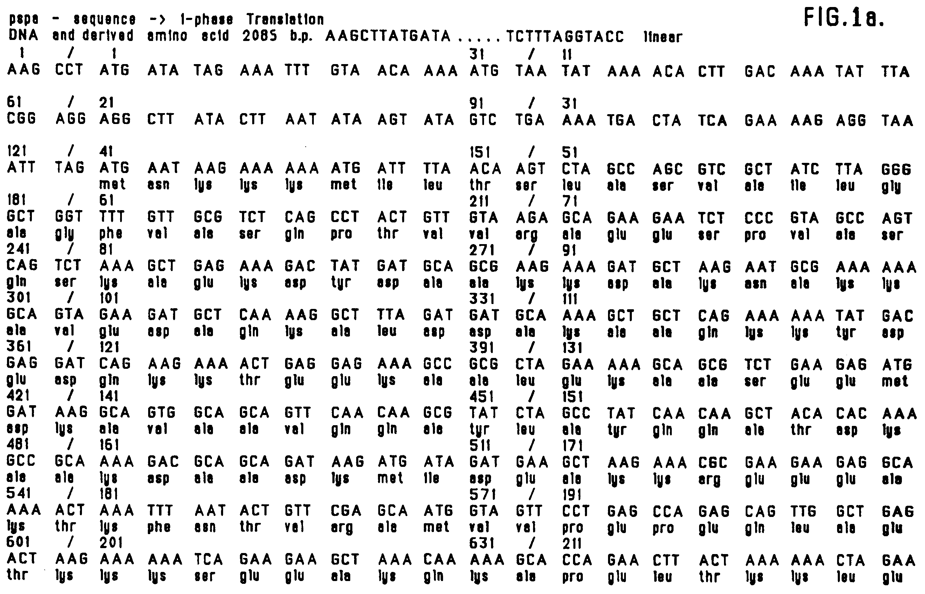

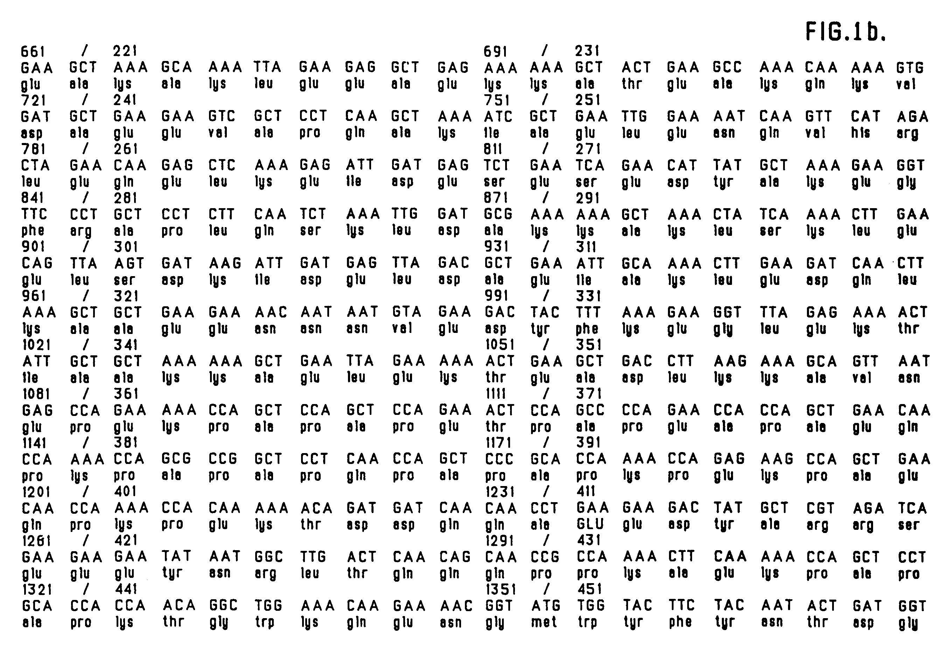

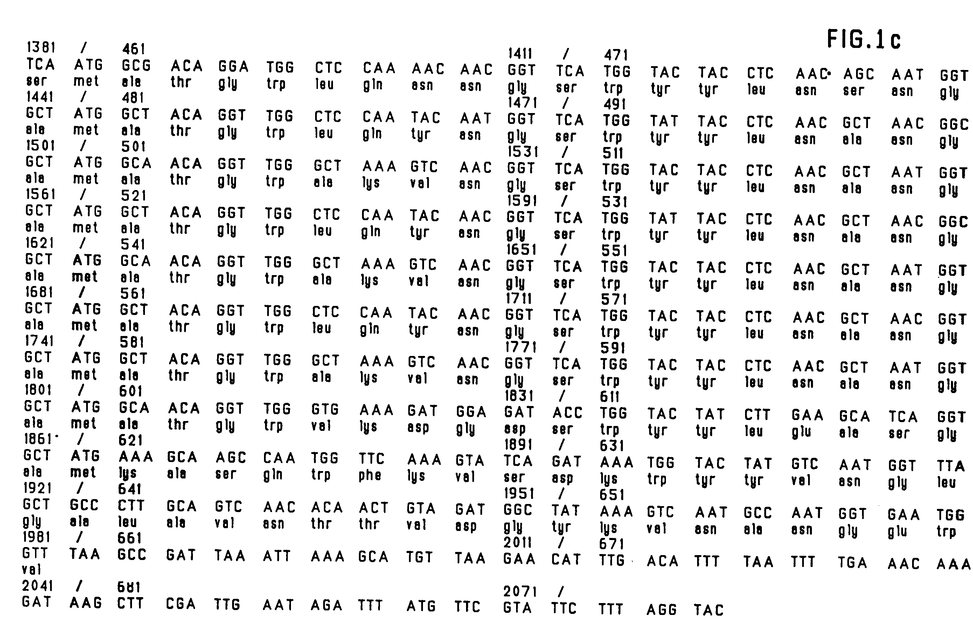

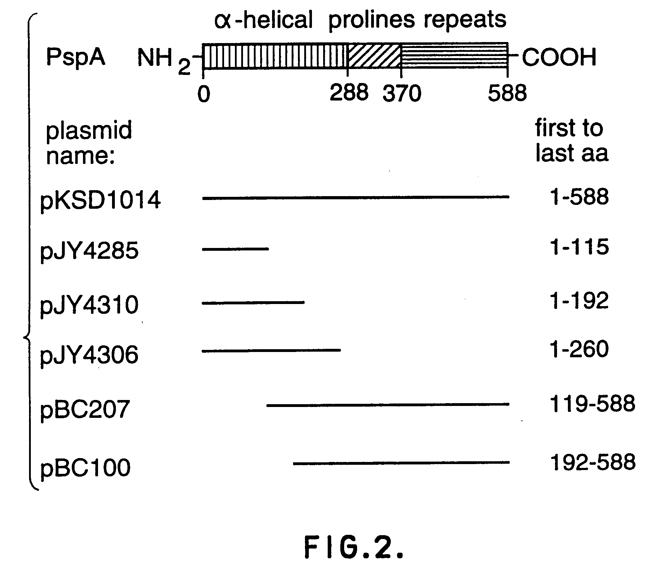

As described in the prior U.S. Patent applications referred to above and in Yother et al (I) and (II), the pspA gene of strain Rx1 encodes a 65 kDa molecule composed of 588 amino acids. The nucleotide sequence (SEQ ID No: 1) of the psaA gene and derived amino acid sequence (SEQ ID No: 2) are set forth in Figure 1. The N-terminal half of the molecule is highly charged and its DNA sequence predicts an $\alpha$-helical coiled-coil protein structure for this region (288 amino acids), as seen in Figure 2. The C-terminal half of PspA, which is not $\alpha$-helical, includes a proline-rich region (83 amino acids) and a repeat region containing the highly conserved twenty amino acid repeats, as well as a slightly hydrophobic sequence of 17 amino acids at the C-terminus. It is known that PspA is anchored to S. pneumoniae by its C-terminal half and it is likely that the proline-rich region serves to tangle the molecule in the cell wall. In addition, it is anticipated that the highly-charged $\alpha$-helical region begins at the cell wall and extends into and possibly through the capsule. This model is supported by the observation that the $\alpha$-helical domain contains all the surface exposed epitopes recognized by monoclonal antibodies (MAbs) reactive with PspA on the pneumococcal surfaces.

The PspA protein of S. pneumoniae strain Rx1 has been mapped to locate protection-eliciting epitopes. Such mapping has been effected by employing antibodies to PspA protein and recombinant fragments of PspA. This mapping technique, described in detail in the Examples below, has identified an amino acid sequence corresponding to the C-terminal third of the $\alpha$-helical region of PspA as containing protection-eliciting epitopes, specifically the amino acid residues 192 to 260 of the Rxl PspA protein. The amino acid sequence from residues 192 to 260 is the C-terminal third of the $\alpha$-helical sequence, expected to be near the cell wall surface.

Since the portion of the sequence from residues 192 to 260 contains only 68 amino acids, individual PspA protein fragments of this size may not be optimally antigenic. This difficulty is overcome by producing recombinant proteins containing tandem fragments of different PspAs expressed by gene fusions of the appropriate portions of several pspA genes.

Accordingly, in a further aspect of the invention, there is provided a PspA protein fragment comprising a plurality of conjugated molecules, each molecule comprising amino acid residues 192 to 260 of the PspA protein of the Rxl strain of Streptococcus pneumoniae and containing at least one protection-eliciting epitope, each molecule being derived from a different strain of S. pneumoniae.

Such tandem molecules can be engineered to maintain proper coiled-coil structure at the points of junction and to be large enough to be immunogenic and to express an array of protection-eliciting epitopes that may cross-react with a wide spectrum of PspAs. Alternatively, individual recombinantly-produced peptides may be attached by chemical means to form a complex molecule.

A further alternative is to attach the PspA fragment to a larger carrier protein or bacterial cell, either as a recombinant fusion product or through chemical attachment, such as by covalent or ionic attachment.

The protein fragments and peptide analogs thereof provided herein are useful components of a vaccine against disease caused by pneumococcal infection. Accordingly, the present invention provides, in a yet further aspect, a vaccine comprising the PspA protein fragments defined herein as an immunologically-active component thereof.

4

BIOLOGICAL MATERIALS

The Examples which follow as well as in the accompanying drawings, reference is made to certain plasmid materials containing whole or truncated pspA gene sequences. The following Table II provides a summary of such materials:

Table II

| Plasmid | Identification | Gene Product |
| --- | --- | --- |
| pKSDIOI4 | whole gene | amino acids 1 to 588 |
| pJY4284 or pJY4285 | 5' terminal region | amino acids 1 to 115 |
| pJY43IO | 5'-terminal region | amino acids 1 to 192 |
| pJY43O6 | 5'-terminal region | amino acids 1 to 260 |
| pBC2O7 | 3'-terminal region | amino acids 119 to 588 |
| pBCIOO | 3'-terminal region | amino acids 192 to 588 |

EXAMPLES

Example 1:

This Example describes the bacterial strains, plasmids and hybridoma antibodies used herein.

S. pneumoniae strains, identified in Table III below, were grown in Todd Hewitt broth with 0.5% yeast extract at 37°C or on blood agar plates containing 3% sheep blood in a candle jar. E. coli strain DHI (Hanahan, J. Mol. Biol. 166:557) was grown in LB medium or minimal E medium. Plasmids included pUCl8 (Gene 33:103), pJY4l63 (Yother et al (II)), and pIN-III-ompA (EMBO J. 3:2437).

All antibody-secreting hybridoma lines were obtained by fusions with non-antibody-secreting myeloma cell line P3-X63-Ag.8.653 (J. Immunol. 123:1548). The specific antibodies employed are identified in Table III below. The anti-PspA hybridoma cell lines Xi64, Xil26 and XiR278 have previously been described in McDaniel et al (I) and Crain et al (supra) . The remaining cell lines were prepared by immunising CBA/N mice with recombinant D39 PspA expressed in λgtII by the technique described in McDaniel et al (I). The cell lines producing antibodies to PspA were all identified using an ELISA in which microtitration plates were coated with heat-killed (60°C, 30 mins) S. pneumoniae R36A or Rxl, which would select for those MAbs that react with surface exposed epitopes on PspA. The heavy chain isotypes of the MAbs were determined by developing the ELISA with affinity purified goat antibody specific for μ and γ heavy chains of IgM and IgG mouse immunoglobulin. The specificity of the MAbs for PspA was confirmed by immunoblot analysis.

All six newly-produced MAbs, identified in Table III as XiR 1526, XiR 35, XiR 1224, XiR 16, XiR 1325 and XiR 1323, detected a protein of the expected size (apparent molecular weight of 84 kDa) in an immunoblot of strains Rxl and D39. No reactivity was observed for any of the MAbs in an immunoblot of strain WG44.1, a PspA-variant of Rxl (see McDaniel et al (III) and Yother et al (II)).

## TABLE III

| Reactivities of MAbs with PspAs from *Streptococcus pneumoniae* | | | | | | | | | | | | |
|---|---|---|---|---|---|---|---|---|---|---|---|---|
| *Streptococcus pneumoniae* | | | | Monoclonal Antibody (Isotype) | | | | | | | | |
| Strain | Capsule type | PspA type | Ref. # | XJR1526 (IgG2b) | XJR35 (IgG2a) | XJR1224 (IgM) | Xi126 (IgG2b) | XiR16 (IgG2a) | Xi64 (IgM) | XJR1325 (IgG2a) | XJR278 (IgG1) | XJR1323 (IgM) |
| Rx1 | rough | 25 | 36 | ++ | ++ | ++ | ++ | ++ | ++ | ++ | ++ | ++ |
| ATCC101813 | 3 | 3 | 37 | - | - | - | ++ | - | ++ | ++ | ++ | ++ |
| EF10197 | 3 | 18 | 38 | - | - | - | - | - | - | -/+ | ++ | - |
| BG9739 | 4 | 26 | 38 | - | - | - | - | - | - | ++ | + | ++ |
| L81905 | 4 | 23 | 38 | - | - | - | - | - | - | - | - | - |
| BG-5-8A | 6A | 0 | 38 | - | - | + | -/+ | - | - | - | + | + |
| BG9163 | 6B | 21 | 38 | - | - | - | - | - | - | - | + | - |
| LM100 | 22 | ND | • | - | - | - | -/+ | - | - | - | - | - |
| WU2 | 3 | 1 | 39 | - | - | - | ++ | - | ++ | ++ | ++ | ++ |
| Protection against WU2 | | | | - | - | - | + | - | + | + | + | + |

EP 0 622 081 A2

Example 2:

This Example describes the provision of the pspA gene from pneumococcal strain Rxl by polymerase chain reaction (PCR).

PCR primers were designed based on the sequence of the pspA gene from pneumococcal strain Rx1 (see Figure 1). The 5'-primers were LSM3 and LSM4. LSM3 was 28 bases in length and started at base 576 and LSM4 was 31 bases in length and started at base 792, and both contained an additional BamHI site. The 3' pspA primer was LSM2 which was 33 bases in length and started at base 1990 and contained an additional SalI site.

The nucleotide sequences for the primers are as follows

LSM2    5'-GCGCGTCGACGGCTTAAACCCATTCACCATTGG-3'    (SEQ ID NO:3)

LSM3    5'-CCGGATCCTGAGCCAGAGCAGTTGGCTG-3'    (SEQ ID NO:4)

LSM4    5'-CCGGATCCGCTCAAAGAGATTGATGAGTCTG-3'    (SEQ ID NO:5)

Approximately 10 ng of genomic Rxl pneumococcal DNA was amplified using a 5' and 3' primer pair. The sample was brought to a total volume of 50 μl containing a final concentration of 50 mM KCl, 10 mM tris-HCl (pH 8.3), 1.5 mM MgCl$_2$, 0.001% gelatin, 0.5 mM each primer and 200 mM of each deoxy-nucleoside triphosphate and 2.5 U of Taq DNA polymerase. Following overlaying of the samples with 50 μl of mineral oil, the samples were denatured at 94°C for 2 mins and then subjected to 10 cycles consisting of 1 min. at 94°C, 2 min. at 50°C and 3 min. at 72°C, followed by another 20 cycles of 1 min. at 94°C, 2 min. at 60°C and 3 min. at 72°C. After completion of the 30 cycles, the samples were held at 72°C for an additional 5 min., prior to cooling to 4°C.

Example 3:

This Example describes expression of truncated PspA molecules.

3'-deleted pspAs that express N-terminal fragments in E. coli and which secrete the same fragments from pneumococci were constructed as described in the aforementioned U.S. patent applications Serial Nos. 835,698 and 656,773 (see also Yother et al (II), supra).

For expression of 5'-deleted pspA constructs, the secretion vector pIN-III-ompA was used. Amplified psaA fragments were digested with BamHI and SalI and ligated into the appropriately BamHI/SalI- digested pIN-III-ompA vector, providing the inserted fragment fused to the ompA leader sequence in frame and under control of the lac promoter. Transformants of E. coli DHI were selected on minimal E medium supplemented with casamino acids (0.1%), glucose (0.2%) and thiamine (0.05 mM) with 50 μg/ml of ampicillin.

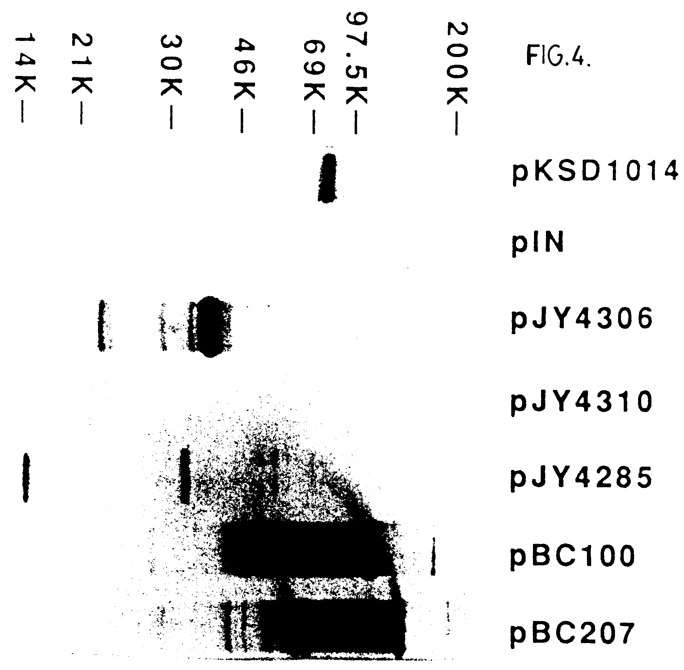

For induction of lac expression, bacteria were grown to an optical density of approximately 0.6 at 660 nm at 37°C in minimal E medium and IPTG was added to a concentration of 2 mM. The cells were incubated for an additional two hours at 37°C, harvested and the periplasmic contents released by osmotic shock. An immunoblot of the truncated PspA proteins produced by the various plasmids is shown in Figure 4.

By these procedures, there were provided, for the 3'-deleted pspAs, plasmids pJY4284, pJY4285, pJY43l0 and pJY4306 and for the 5'-deleted pspAs, plasmids pBC207 and PBC100. Plasmid pJY4284 and pJY4285 contain an insert of 564 base pairs, nucleotides 1 to 564 and encoded a predicted 13 kDa PspA C-terminal-deleted product corresponding to amino acids 1 to 115. Plasmid pJY43l0 contains an insert of 795 base pairs, nucleotides 1 to 795 and encoded a predicted 21 kDa C-terminal-deleted product corresponding to amino acid 1 to 192. However pJY4306 contained an insert of 999 base pairs, nucleotides 1 to 999 and encoded a predicted 29 kDa C-terminal-deleted product corresponding to amino acids 1 to 260. Plasmid pBC100 contained an insert of 1199 base pairs, nucleotides 792 to 1990, and encoded a predicted 44 kDa PspA N-terminal deleted product containing amino acids 192 to 588. pBC207 contained an insert of 1415 base pairs, nucleotide 576 to 1990, and encoded a predicted 52 kDa PspA N-terminal deleted product containing amino acids 119 to 588.

The pspA gene sequences contained in these plasmids code for and express amino acids as identified in Figure 2.

Example 4:

This Example describes the procedure of effecting immunoassays.

Immunoblot analysis was carried out as described in McDaniel et al (IV). The truncated PspA molecules prepared as described in Example 3 or pneumococcal preparations enriched for PspA (as described in McDaniel et al (II)) were electrophoresed in a 10% sodium dodecyl sulfate polyacrylamide gel and electro-blotted onto nitrocelluloses. The blots were probed with individual MAbs, prepared as described in Example 1.

A direct binding ELISA procedure was used to quantitatively confirm reactivities observed by immunoblotting. In this procedure, osmotic shock preparations were diluted to a total protein concentration of 3 $\mu$g/ml in phosphate buffered saline (PBS) and 100 $\mu$l was added to wells of Immulon 4 microtitration plates. After blocking with 13 bovine serum albumin in PBS, unfractionated tissue culture supernates of individual MAbs were titered in duplicate by 3-fold serial dilution through 7 wells and developed as described in McDaniel et al (IV) using a goat anti-mouse immunoglobulin alkaline phosphate conjugated secondary antibody and alkaline phosphate substrate. Plates were read in a Dynatech plate reader at 405 nm, and the 30% end point was calculated for each antibody with each preparation.

The protective capacity of the MAbs was tested by injecting three CBA/N mice i.p. with 0.1 ml of 1/10 dilution (about 5 to 30 $\mu$g) of each hybridoma antibody 1 hr prior to i.v. injection of $10^3$ CFU of WU2 or D39 pneumococci (>100 x $LD_{50}$). Protection was judged as the ability to prevent death of all mice in a group. All non-protected mice died of pneumococcal infection within 48 hours post challenge.

Example 5:

This Example describes mapping of the epitopes on PspA using the monoclonal antibodies described in Example 1.

The six newly-produced monoclonal antibodies described in Example 1 and identified in Table III were used along with the previously-described monoclonal antibodies Xi64, Xi126 and XiR278 to map epitopes on PspA.

To determine whether each of the MAbs recognized different epitopes, each of them was reacted with eight additional S. pneumoniae strains, as identified in Table III, in immunoblots of SDS-PAGE separated proteins. Seven different patterns of activity were observed. Three antibodies, XiRI6, XiR35 and XiRI526, appeared to recognize epitopes found on Rxl PspA but none of the other PspAs. Accordingly, it was possible that these three antibodies might all react with the same epitope as Rxl PspA.

MAb Xi64 and Xi126 both reacted strongly only with epitopes on ATCC 101813, WU2 and Rxl PspAs, but not with PspAs of the other strains. However, it is known from studies of larger panels of PspAs (as described in McDaniel et al (III) and Crain et al) that Xi126 and Xi64 recognize different determinants.

The remaining four antibodies each exhibited unique patterns of reactivity with the panel of PspAs. Accordingly, the nine antibodies tested recognized at least seven different epitopes on PspA.

For reasons which are not clear, the type 2 strain D39 appeared to be uniquely able to resist the protective effects of antibodies to PspA (McDaniel et al (IV)). As described in McDaniel et al (I), greater than forty times the amount of Xi126 was required to passively protect against the D39 strain as compared to the WU2 strain. None of the six newly-produced monoclonal antibodies protected against the D39 strain. In contrast, immunization of mice with Rxl PspA elicits protection against A66, WU2 and EF6796 strains (mouse virulent pneumococci of capsular types 3, 3 and 6A respectively), all of which have PspA types that are different from those of Rxl and D39 (see McDaniel et al (IV)). In view of the close serologic similarity between the type 25 PspA of Rxl and type 1 PspA of WU2 (Crain et al), WU2 pneumococci were used to challenge mice that had been passively protected with the MAbs. All five of the MAbs that were observed to bind WU2 PspA were able to protect against infection with 1000 CFU of WU2. Protective antibodies were found in IgM, IgGl, IgG2b and Ig2a heavy chain isotype classes.

Example 6:

This Example describes mapping of the epitopes of PspA using the recombinant truncated PspA molecules formed in Example 3.

The five-overlapping C-terminal or N-terminal deleted PspA fragments, prepared as described in Example 3 and shown in Figure 2, were used to map epitopes on PspA. The general location of the epitopes detected by each of the mice MAbs, as described in Example 5, was determined using the five C-terminal-deleted and two N-terminal deleted PspA molecules. As a positive control, the reactivity of each antibody was examined with a clone, pKSDI0I4, expressing full-length PspA.

As noted earlier, the reactivity of the MAb was determined by two methods. In one method, reactivity between the fragments and MAb was evaluated in immunoblots of the fragment preparations after they had been separated by SDS-PAGE. In the second method, a direct ELISA was used to quantify the reactivity of the MAbs with non-denatured PspA fragment.

The reactivities observed and the quantification of such activity is set forth in the following Table IV:

8

Table IV: Reactivity of PspA Fragments with Monoclonal Antibodies[1]

| PspA Fragments | Monoclonal Antibodies | | | | | | | | |
|---|---|---|---|---|---|---|---|---|---|
| | Xi126 | XIR35 | XIR1526 | XIR1224 | XIR16 | XIR1323 | X164 | XIR1325 | XIR278 |
| pJY4285 | + + 72 | + + 5 | + + <3 | + <3 | + 4 | - <3 | - <3 | - <3 | - <3 |
| pJY4310 | + + 116 | + + 4 | + + <3 | + 5 | + + 16 | - 31 | - <3 | - <3 | - <3 |
| pJY4306 | + + 1127 | + + 78 | + + 554 | + + 805 | + + 2614 | + + <3 | + + 643 | + + 717 | + <3 |
| pBC207 | - <3 | - <3 | - <3 | - <3 | + <3 | + + 61 | + + <3 | + + <3 | + + 4527 |
| pBC100 | - <3 | - <3 | - <3 | - <3 | - <3 | + + 15 | + + 709* | + + 4401 | + + 4746 |
| Rxl | + + 63 | + + 15 | + + 42 | + + 48 | + + 118 | + + 44 | + + 64 | + + 111 | + + 468 |
| pIN-III | - <3 | - <3 | - <3 | - <3 | - <3 | - <3 | - <3 | - <3 | - <3 |

1. Antibodies were reacted with the indicated PspA fragments in immunoblot of SDS-PAGE separations, or by ELISA using microtitration plates coated with preparations enriched for the indicated PspA fragments. Rxl PspA serves as a positive control, and pIN-III-ompA (vector alone) serves as a negative control. The results of the immunoblot are presented as ++ (strong reaction), + (weak but clearly positive reaction) and - (no reaction). ELISA values are given as the reciprocal dilution of each monoclonal antibody that gave 30% of maximum binding with wells coated with the indicate fragment preparation.

The asterisk (*) after some of the antibodies denotes those which are able to protect against fatal pneumococcal infection with strain WU2 or D39 S. pneumoniae.

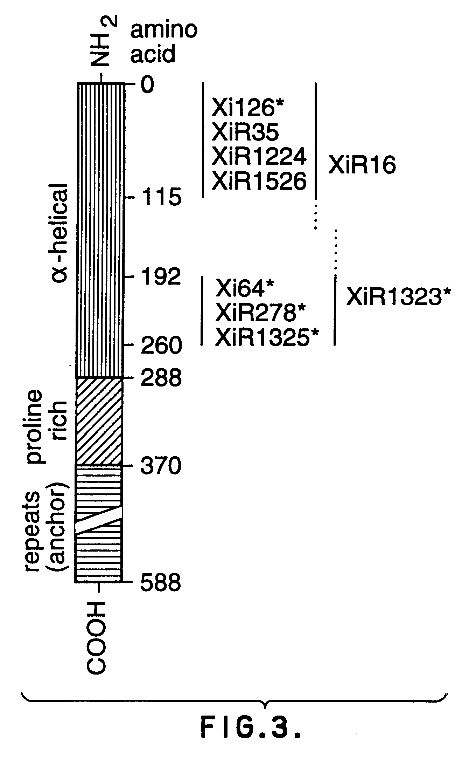

The deduced locations of the epitopes are indicated in Figure 3.

As can be seen from the data in Table IV, three of the antibodies, Xil26 and XiR35 and XiRl526, react strongly with all three C-terminal-deleted clones in immunoblot analysis, indicating that the sequence required to form the epitope(s) detected by all three lies within the first 115 amino acids of PspA. This map position is in agreement with the failure of these antibodies to react with either of the N-terminal-deleted clones that lack the first 119 and 191 amino acids.

MAb XiRl224 reacted strongly by immunoblot with the longest C-terminal-deleted fragment (pJY4306), but showed substantially weaker reactions with the shorter two C-terminal-deleted fragments. This result indicates that, while the binding site of the antibody may be in the first 115 amino acids, residues beyond amino acid 192 may be important for the conformation or stability of the epitope.

By immunblot, the three antibodies Xi64, XiRl325 and XiR278, all reacted with the longest C-terminal-deleted fragment and both of the N-terminal-deleted fragments, thus locating their determinants between amino acid positions 192 and 260. Generally confirmatory results were obtained in ELISAs with the native molecules. However, in a few cases, reactions were observed in ELISAs with full length PspA but not with a truncated molecule even though the same truncated fragment was reactive with the antibody by immunoblot. These observations may have resulted from an altered conformation of the truncated fragments under physiologic conditions that masked or prevented the formation of determinant present in full-length PspA and in the denatured fragments.

Two antibodies XiR2l6 and XiRl323 showed what, at first appeared to be anomalous reactions, indicating that epitopes detected by the antibodies might be in more than one portion of PspA. In view of this unexpected result, the assays were repeated multiple times with two sets of preparations of the truncated fragments. The results of the additional assays confirmed the two-position mapping of epitopes for these two MAbs.

By immunoblot, MAb XiR16 reacted strongly with the two longest C-terminal-deleted fragments and failed to react with the shortest N-terminal-deleted fragment. Accordingly, the epitope detected must be N-terminal to position 192. Unexpectedly, Mab XiR16 reacted weakly in immunoblots with both the longest N-terminal-deleted fragment (residues 119 to 158) and the shortest C-terminal-deleted fragment (residues 1 to 115). Since the fragments do not overlap, and if the weak immunoblot reactivities with fragments (reactivities not seen by ELISA) are not an artifact, the MAb XiRl6 must recognize epitopes on both fragments.

In the case of MAb XiRl323, the immunoblot data clearly places the detected epitope between positions 192 and 260. In the ELISA studies, however, XiRl323 reacted strongly and reproducibly with the C-terminal-deleted fragment pJY4310 (amino acid residues 1 to 192) as well as the shortest N-terminal-deleted fragment pBCl00 (amino acid residues 192 to 588). Curiously, an ELISA reaction was not observed between MAb XiRl323 and pJY4306 (amino acid residues 1 to 260), even though MAb XiRl323 reacted strongly with this fragment by immunoblot.

These findings provide additional evidence for distal conformation effects on antigenic determinants of PspA. They also indicate that, on the native fragments, MAb XiRl323 sees epitopes on both sides of position 192. The relationship between expression of the epitopes in other PspAs and their position in Rxl PspA is demonstrated in Table IV in which is listed the antibodies in accordance with their apparent map position in PspA. The five antibodies (including XiRl6) that clearly recognize epitopes N-terminal to position 116 are listed at the left side of Table IV. The four antibodies that clearly recognize epitopes C-terminal to position 192 are listed on the right side of Table IV. Three of the five epitopes N-terminal of position 192 (those recognized by XiRl526, XiR35, and XiR16) were not found on any of the other eight PspAs tested. One epitope (recognized by XiR 1224) was weakly expressed by one other strain and another (recognized by Xil26) was expressed on two other strains. In contrast, the four epitopes present in the C-terminal third of the PspA $\alpha$-helical region were each present in from two to six other strains. The greater conservation of the region C-terminal to position 192, as compared to the region N-terminal to position 192 was significant at $P<0.05$ by both the Chi-square and the two sample rank tests. Based on the mapping results (Table III) and the strain distribution results (Table IV), it is apparent that all of the antibodies except possibly XiR35 and XiRl526 must recognize different PspA determinants.

Example 7:

This Example contains a discussion of the mapping results achieved in Example 6.

The results set forth in Example 6 clearly demonstrate that the protection eliciting epitopes of PspA are not restricted to the N-terminal end of the surface exposed $\alpha$-helical half of the molecule. In fact, four of the five antibodies protective against S. pneumoniae WU2 reacted with the C-terminal third of the $\alpha$-helical region of PspA. This portion of the $\alpha$-helical region is thought to closest to the cell wall (see Yother et al (II)).

About half of the MAbs recognized determinants N-terminal to amino acid 115 and the other half recognized epitopes C-terminal to residue 192. Since the nine antibodies were selected for their ability to bind native PspA on the surface of heat-killed whole pneumococci, the distribution of the epitopes they recognize suggests that determinants between positions 115 and 192 are either not immunogenic or are not exposed on the native molecule as expressed on pneumococci.

Curiously two MAbs (XiRI6 and XiRI323) appeared to possibly react with epitopes in more than one position on PspA. Although the bulk of the data for XiRI6 placed its epitope N-terminal of position 115, weak immunoblot patterns suggested that a reactive epitope(s) may also exist C-terminal to residue 115. In the case of XiRI323, the bulk of the data indicated that its epitope is between positions 192 and 260. However, the ELISA assay showed significant reactivity of the antibody with a C-terminal-deleted PspA fragment extending from residues 1 to 192. Although there are no extensive repeats in the N-terminal half of PspA, there are a few short repeated sequences that occur more than once in the coiled-coil motif. One such sequence is glu-glu-ala-lys which starts at amino acid positions 105, 133, and 147 and another is lys-ala-lys-leu starting at positions 150 and 220 (see Figure 1). In the case of XiRI323, the antibody reacted with the epitope on the 1 to 192 fragment under natured but not denatured conditions. This may indicate that the epitope is conformational and may not have the same exact sequence as the epitope recognized (under both natured and denatured conditions) between residues 192 and 260.

One mechanism that may account for the lack of exposure of epitopes between amino acid 115 and 192 would be a folding back of this portion of the $\alpha$-helical sequence on itself or other parts of PspA to form a coiled-coil structure more complex than a simple coiled-coil dimer. If this occurred, it could explain how PspA tertiary structure can sometimes be dependent on distant PspA structures. A suggestion that this might, in fact, be the case comes from the observation that some of the truncated forms did not express certain epitopes under physiologic conditions that were detected on the whole molecule under the same conditions and were shown to be present in the fragment after denaturation in SDS.

Since a PspA vaccine may need to contain fragments of several serologically different PspAs, it would be desirable to include in a vaccine only those portions of each PspA that are most likely to elicit cross-protective antibodies. Based on the results presented herein with Rxl PspA, it appears likely that the portion of the PspA sequences corresponding to residues 192 to 260 of Rxl PspA is the best portion of PspA to include in a recombinant PspA vaccine. The epitopes in this portion of PspA were three and a half times as likely to be present in the PspAs of other strains as the epitopes in the residue 1 to 115 portion of the sequence, and none of the 9 antibodies studied clearly reacted with the middle third of the $\alpha$-helical region.

## Example 8:

This Example shows protection of mice by PspA fragments. Five mice were immunized with purified fragment produced by pBC207 in E. coli and five with purified fragment produced by pBCl00 in E. coli. In both cases, the fragments were injected in Freund's complete adjuvant. All mice immunized with each fragment survived challenge with 100 x $LD_{50}$ of WU2 capsular type 3 S. pneumoniae.

Five additional mice were injected with adjuvant plus an equivalent preparation on non-PspA producing E. coli. All mice died when challenged with the same dose of WU2.

The data presented in this Example conclusively proves that epitopes C-terminal to amino acids 119 and 192 respectively are capable of eliciting protective immunity. This result is consistent with the findings presented in the earlier Examples that the region of PsPa from amino acids 192 to 260 contain protection-eliciting epitopes.

## SEQUENCE IDENTIFICATIONS

SEQ ID NO: 1 DNA sequence for pspA gene (Figure 1)

SEQ ID NO: 2 Deduced amino acid sequence for PspA protein (Figure 1)

SEQ ID NO: 3 Nucleotide sequence for PCR primer LSM 2

SEQ ID NO: 4 Nucleotide sequence for PCR primer LSM 3

SEQ ID NO: 5 Nucleotide sequence for PCR primer LSM 4

In summary of this disclosure, the present invention provides a PspA protein fragment which contains protection-eliciting epitopes and which is cross-reactive and can be incorporated into a vaccine against disease caused by pneumococcal infection. Modifications are possible within the scope of this invention.

The term "effectively homologous" used herein means in relation to an amino acid sequence effectively homologous to a defined sequence, that the said amino acid sequence may not be identical to said defined sequence but may be at least 70 percent, more preferably 80 percent, still more preferably 90 percent identical, provided that the antigenic epitope or epitopes in said amino acid sequence have properties substantially the same as the corresponding epitopes in said defined sequence.

Now that the region constituted by residues 192 to 260 in the PspA protein of the Rx1 strain has been identified, those skilled in the art will readily be able to produce by recombinant techniques protein fragments according to the invention. In particular, they may tailor DNA probes to use in a PCR reaction to amplifying genomic DNA coding for a desired fragment, insert the amplified DNA into a suitable plasmid vector and utilise the vector in a known manner to express the protein in a suitable host such as E. coli, adapting the methods taught in Example 3 above.

It will be possible to clone and express the appropriate pspA fragments and express their truncated products under the control of an appropriate promoter, e.g. a vector containing the E.coli lac promoter expressing the E. coli ompA and leader sequence to create an ompA::pspA fusion plasmid. Optionally, the sequence coding for the PspA fragment may be linked to a sequence coding for a further protein suitable for injection into humans. Such proteins would likely be those already used as vaccines because they are known to elicit protective immune responses and/or known to function as strong immunologic carriers. Such proteins could include the partial or complete amino acid sequence of toxins such as tetanus toxin, or outer membrane proteins such as that of group B subtype 2 Neisseria meningitis.

It will also be possible to produce a fusion protein composed of the cross-reactive protection-eliciting regions of several different PspA molecules. Such a fusion protein could be made large enough ($\geq$40,000 molecular weight) to be highly immunogenic and as a single protein could elicit cross-protection to as many different pneumococci as possible. The combination of cross-protective 70 amino acid regions from 5 to 6 PspAs would be large enough to be highly immunogenic. Constructs expressing epitopes from more than one PspA are especially attractive since PspAs of pneumococci are known to differ serologically. Present evidence indicates that a widely protective vaccine will need to contain cross-reactive protection-eliciting epitopes from more than one different pneumococcus.

It is possible to design such a fusion protein so that it also carries a domain that would help with isolation by including the choline binding region of PspA, or a ligand binding domain from other proteins (such as the maltose binding protein [encoded by malE] of E. coli. In the former case the fusion protein could be isolated by adsorption to a choline Sepharose column and elution using 2% choline chloride. In the latter case adsorption would be to a mannose-Sepharose column followed by elution with a solution containing mannose.

In the construction of such a fusion protein containing tandem cross-reactive coiled-coil PspA regions it will be critical not only that the appropriate open reading frame of each down stream gene fragment be preserved at the junctions of the ligated gene fragments, but that the heptad motif of the coiled-coil amino acid sequence not be disrupted. One way to accomplish the latter would be to construct the gene fusions so that they occur within naturally occurring noncoil-coiled regions found in the $\alpha$-helical domain of PspA. In our previous report (Yother and Briles J. Bact. T/4:601-609) such non-coiled-coil breaks were identified at amino acid positions 169-176, 199, 225, 254, 274 and 289. Fusions between two or more cross-protective regions (residues 192-260) at or near positions 170 or 199 at one end and at or near residues 274 or 289 at the other end, would be expected to very likely be able to express the epitopes normally expressed within the coiled-coil regions.

In each case, the easiest way to prepare such constructs would be by PCR amplification of the DNA used to construct the gene fusions. In this way it will be possible to prepare the relevant sequence with appropriate restriction sites. The design of gene fusions and the PCR primers used to produce the individual pspA fragments will be carried out so that the proper reading frame will be preserved in each fused gene fragment at the nucleotide level.

It is also possible to synthesise peptides according to the invention having the appropriate amino acid sequence by conventional peptide synthesis.

## Claims

1. An isolated pneumococcal surface protein A (PspA) protein fragment comprising amino acid residues corresponding to all or some of amino acid residues 192 to 260 of the PspA protein of the Rx1 strain of Streptococcus pneumoniae containing at least one protection-eliciting epitope and optionally up to a further 25 resides of said protein in the $NH_2$ terminal direction and/or the COOH terminal direction, or being effectively homologous with such a protein fragment.

2. A protein fragment as claimed in Claim 1, containing an amino acid sequence corresponding to amino acid residues 192 to 260 of the PspA protein of the Rx1 strain.

3. A protein fragment as claimed in Claim 2, having said amino acid sequence.

4. A protein fragment as claimed in Claim 1, 2 or 3 containing an amino acid sequence effectively homologous to the amino acid residues 192 to 260 of the PspA protein of said Rx1 strain.

5. A protein fragment as claimed in Claim 4, constituted by an amino acid sequence effectively homologous to the amino acid residues 192 to 260 of the PspA protein of said Rx1 strain.

6. A protein fragment claimed in any one of Claims 1 to 5 which is produced recombinantly.

7. An isolated protein fragment comprising the amino acid sequence of or effectively homologous with that of a protection-eliciting epitope corresponding to an epitope contained in amino acid residues 192 to 260 of the pneumococcal surface protein A (PspA) protein of the Rx1 strain of Streptococcus pneumoniae, and including no more than 25 additional amino acid residues in the $NH_2$ and or the COOH terminal direction.

8. A pneumococcal surface protein A (PspA) protein fragment comprising a plurality of conjugated molecules, each molecule comprising an isolated protein fragment corresponding to or effectively homologous with a protection-eliciting epitope corresponding to an epitope located in residues 192 to 260 of the PspA of strain Rx1 molecules within said plurality optionally being derived from different strains of S. pneumoniae.

9. A vaccine against disease caused by pneumococcal infection, comprising, as an immunologically-active component, a PspA protein fragment as claimed in any one of Claims 1 to 8.

10. A vaccine as claimed in Claim 9, characterised in that said PspA protein fragment is as claimed in any one of Claims 1 to 7 and is conjugated to a larger molecule.

11. A DNA primer or probe having the nucleotide sequence:-

5'-CCGGATCCTGAGCCAGAGCAGTTGGCTG-3'

12. A DNA primer or probe having the nucleotide sequence:-

5'-CCGGATCCGCTCAAAGAGATTGATGAGTCTG-3'

pspe - sequence -> 1-phase Translation
DNA and derived amino acid 2085 b.p. AAGCTTATGATA ..... TCTTTAGGTACC linear

```
1         /  1                                31        /  11
AAG  CCT  ATG  ATA  TAG  AAA  TTT  GTA  ACA  AAA  ATG  TAA  TAT  AAA  ACA  CTT  GAC  AAA  TAT  TTA


61        /  21                               91        /  31
CGG  AGG  AGG  CTT  ATA  CTT  AAT  ATA  AGT  ATA  GTC  TGA  AAA  TGA  CTA  TCA  GAA  AAG  AGG  TAA


121       /  41                               151       /  51
ATT  TAG  ATG  AAT  AAG  AAA  AAA  ATG  ATT  TTA  ACA  AGT  CTA  GCC  AGC  GTC  GCT  ATC  TTA  GGG
          met  asn  lys  lys  lys  met  ile  leu  thr  ser  leu  ala  ser  val  ala  ile  leu  gly
181       /  61                               211       /  71
GCT  GGT  TTT  GTT  GCG  TCT  CAG  CCT  ACT  GTT  GTA  AGA  GCA  GAA  GAA  TCT  CCC  GTA  GCC  AGT
ala  gly  phe  val  ala  ser  gln  pro  thr  val  val  arg  ala  glu  glu  ser  pro  val  ala  ser
241       /  81                               271       /  91
CAG  TCT  AAA  GCT  GAG  AAA  GAC  TAT  GAT  GCA  GCG  AAG  AAA  GAT  GCT  AAG  AAT  GCG  AAA  AAA
gln  ser  lys  ala  glu  lys  asp  tyr  asp  ala  ala  lys  lys  asp  ala  lys  asn  ala  lys  lys
301       /  101                              331       /  111
GCA  GTA  GAA  GAT  GCT  CAA  AAG  GCT  TTA  GAT  GAT  GCA  AAA  GCT  GCT  CAG  AAA  AAA  TAT  GAC
ala  val  glu  asp  ala  gln  lys  ala  leu  asp  asp  ala  lys  ala  ala  gln  lys  lys  tyr  asp
361       /  121                              391       /  131
GAG  GAT  CAG  AAG  AAA  ACT  GAG  GAG  AAA  GCC  GCG  CTA  GAA  AAA  GCA  GCG  TCT  GAA  GAG  ATG
glu  asp  gln  lys  lys  thr  glu  glu  lys  ala  ala  leu  glu  lys  ala  ala  ser  glu  glu  met
421       /  141                              451       /  151
GAT  AAG  GCA  GTG  GCA  GCA  GTT  CAA  CAA  GCG  TAT  CTA  GCC  TAT  CAA  CAA  GCT  ACA  CAC  AAA
asp  lys  ala  val  ala  ala  val  gln  gln  ala  tyr  leu  ala  tyr  gln  gln  ala  thr  asp  lys
481       /  161                              511       /  171
GCC  GCA  AAA  GAC  GCA  GCA  GAT  AAG  ATG  ATA  GAT  GAA  GCT  AAG  AAA  CGC  GAA  GAA  GAG  GCA
ala  ala  lys  asp  ala  ala  asp  lys  met  ile  asp  glu  ala  lys  lys  arg  glu  glu  glu  ala
541       /  181                              571       /  191
AAA  ACT  AAA  TTT  AAT  ACT  GTT  CGA  GCA  ATG  GTA  GTT  CCT  GAG  CCA  GAG  CAG  TTG  GCT  GAG
lys  thr  lys  phe  asn  thr  val  arg  ala  met  val  val  pro  glu  pro  glu  gln  leu  ala  glu
601       /  201                              631       /  211
ACT  AAG  AAA  AAA  TCA  GAA  GAA  GCT  AAA  CAA  AAA  GCA  CCA  GAA  CTT  ACT  AAA  AAA  CTA  GAA
thr  lys  lys  lys  ser  glu  glu  ala  lys  gln  lys  ala  pro  glu  leu  thr  lys  lys  leu  glu
```

EP 0 622 081 A2

FIG.1b.

EP 0 622 081 A2

```
661      /  221
GAA GCT AAA GCA AAA TTA GAA GAG GCT GAG    AAA AAA GCT ACT GAA GCC AAA CAA AAA GTG
glu ala lys ala lys leu glu glu ala glu    lys lys ala thr glu ala lys gln lys val
721      /  241                            751      /  251
GAT GCT GAA GAA GTC GCT CCT CAA GCT AAA    ATC GCT GAA TTG GAA AAT CAA GTT CAT AGA
asp ala glu glu val ala pro gln ala lys    ile ala glu leu glu asn gln val his arg
781      /  261                            811      /  271
CTA GAA CAA GAG CTC AAA GAG ATT GAT GAG    TCT GAA TCA GAA CAT TAT GCT AAA GAA GGT
leu glu gln glu leu lys glu ile asp glu    ser glu ser glu asp tyr ala lys glu gly
841      /  281                            871      /  291
TTC CCT GCT CCT CTT CAA TCT AAA TTG GAT    GCG AAA AAA GCT AAA CTA TCA AAA CTT GAA
phe arg ala pro leu gln ser lys leu asp    ala lys lys ala lys leu ser lys leu glu
901      /  301                            931      /  311
CAG TTA AGT GAT AAG ATT GAT GAG TTA GAC    GCT GAA ATT GCA AAA CTT GAA GAT CAA CTT
glu leu ser asp lys ile asp glu leu asp    ala glu ile ala lys leu glu asp gln leu
961      /  321                            991      /  331
AAA GCT GCT GAA GAA AAC AAT AAT GTA GAA    GAC TAC TTT AAA GAA GGT TTA GAG AAA ACT
lys ala ala glu glu asn asn asn val glu    asp tyr phe lys glu gly leu glu lys thr
1021     /  341                            1051     /  351
ATT GCT GCT AAA AAA GCT GAA TTA GAA AAA    ACT GAA GCT GAC CTT AAG AAA GCA GTT AAT
ile ala ala lys lys ala glu leu glu lys    thr glu ala asp leu lys lys ala val asn
1081     /  361                            1111     /  371
GAG CCA GAA AAA CCA GCT CCA GCT CCA GAA    ACT CCA GCC CCA GAA CCA CCA GCT GAA CAA
glu pro glu lys pro ala pro ala pro glu    thr pro ala pro glu ala pro ala glu gln
1141     /  381                            1171     /  391
CCA AAA CCA GCG CCG GCT CCT CAA CCA GCT    CCC GCA CCA AAA CCA GAG AAG CCA GCT GAA
pro lys pro ala pro ala pro gln pro ala    pro ala pro lys pro glu lys pro ala glu
1201     /  401                            1231     /  411
CAA CCA AAA CCA CAA AAA ACA GAT GAT CAA    CAA CCT GAA GAA GAC TAT GCT CGT AGA TCA
gln pro lys pro glu lys thr asp asp gln    gln ala GLU glu asp tyr ala arg arg ser
1261     /  421                            1291     /  431
GAA GAA GAA TAT AAT GGC TTG ACT CAA CAG    CAA CCG CCA AAA CTT CAA AAA CCA GCT CCT
glu glu glu tyr asn arg leu thr gln gln    gln pro pro lys leu gln lys pro ala pro
1321     /  441                            1351     /  451
GCA CCA CCA ACA GGC TGG AAA CAA GAA AAC    GGT ATG TGG TAC TTC TAC AAT ACT GAT GGT
ala pro lys thr gly trp lys gln glu asn    gly met trp tyr phe tyr asn thr asp gly
```

FIG.1c

```
1381  TCA   /    461              GGA   TGG   CTC   CAA   AAC   AAC   TAC   TAC  1411  /    471   TAC   CTC   AAC   AGC   AAT   GGT
      ser  ATG  GCG   ACA   GGA   gly   trp   leu   gln   asn   asn   tyr   tyr  GGT  TCA  TGG   tyr   leu   asn   ser   asn   gly
           met  ala   thr                                                        gly  ser  trp

1441  GCT   /    481              GGT   TGG   CTC   CAA   TAC   AAT   TAT   TAC  1471  /    491   TAT   CTC   AAC   GCT   AAC   GGC
      ala  ATG  GCT   ACA   GGT   gly   trp   leu   gln   tyr   asn   tyr   tyr  GGT  TCA  TGG   tyr   leu   asn   ala   asn   gly
           met  ala   thr                                                        gly  ser  trp

1501  GCA   /    501              GGT   TGG   GCT   AAA   GTC   AAC   TAC   TAC  1531  /    511   TAC   CTC   AAC   GCT   AAT   GGT
      ala  ATG  GCA   ACA   GGT   gly   trp   ala   lys   val   asn   tyr   tyr  GGT  TCA  TGG   tyr   leu   asn   ala   asn   gly
           met  ala   thr                                                        gly  ser  trp

1561  GCT   /    521              GGT   TGG   CTC   CAA   TAC   AAC   TAC   TAC  1591  /    531   TAC   CTC   AAC   GCT   AAC   GGC
      ala  ATG  GCT   ACA   GGT   gly   trp   leu   gln   tyr   asn   tyr   tyr  GGT  TCA  TGG   tyr   leu   asn   ala   asn   gly
           met  ala   thr                                                        gly  ser  trp

1621  GCA   /    541              GGT   TGG   GCT   AAA   AAC   AAC   TAC   TAC  1651  /    551   TAC   CTC   AAC   GCT   AAT   GGT
      ala  ATG  GCA   ACA   GGT   gly   trp   ala   lys   asn   asn   tyr   tyr  GGT  TCA  TGG   tyr   leu   asn   ala   asn   gly
           met  ala   thr                                                        gly  ser  trp

1681  GCT   /    561              GGT   TGG   CTC   CAA   TAC   AAC   TAC   TAC  1711  /    571   TAC   CTC   AAC   GCT   AAC   GGT
      ala  ATG  GCT   ACA   GGT   gly   trp   leu   gln   tyr   asn   tyr   tyr  GGT  TCA  TGG   tyr   leu   asn   ala   asn   gly
           met  ala   thr                                                        gly  ser  trp

1741  GCT   /    581              GGT   TGG   GCT   AAA   GTC   AAC   TAC   TAC  1771  /    591   TAC   CTC   AAC   GCT   AAT   GGT
      ala  ATG  GCT   ACA   GGT   gly   trp   ala   lys   val   asn   tyr   tyr  GGT  TCA  TGG   tyr   leu   asn   ala   asn   gly
           met  ala   thr                                                        gly  ser  trp

1801  GCA   /    601              GGT   TGG   GTG   AAA   GAT   AAC   TAC   TAC  1831  /    611   TAT   CTT   GAA   GCA   TCA   GGT
      ala  ATG  GCT   ACA   GGT   gly   trp   val   lys   asp   asn   tyr   tyr  GGT  ACC  TGG   tyr   leu   glu   ala   ser   gly
           met  ala   thr                                                        gly  ser  trp

1861  GCT   /    621              AGC   CAA   TTC   AAA   GAT   GGA   TGG   AAA  1891  /    631   TGG   CTT   GTC   AAT   GGT   TTA
      ala  ATG  GCA   GCA   AGC   ser   gln   phe   lys   asp   gly   trp   lys  GAT  GAT  AAA   trp   leu   val   asn   gly   leu
           met  ala                                                              asp  asp  lys

1921  GCT   /    641              GTC   AAC   ACT   ACA   GTA   GTA   AAA   TCA  1951  /    651   GTC   TAT   AAT   AAT   GAA   TGG
      ala  AAA  AAA   GCA   GTC   val   asn   thr   thr   val   val   lys   ser  TCA  GAT  AAA   val   tyr   asn   asn   glu   trp
           lys  lys                                                              ser  asp  lys

1981  GCC   /    661              GTC   AAC   ACA   GTA   GAT   GTC   AAT   GGT  2011  /    671   GTC   GCC   AAT   AAC
      gly  CTT  CTT   GCC   GTC   val   asn   thr   val   asp   val   asn   gly  GGC  TAT  AAA   val   ala   asn   asn
           leu  leu

      GTT        681
      val  TAA  CTT   GAT   TAA   ATT   AAA   TGT   TAA                 ACA   TTG  GAA   CAT  TTG  ACA  TTT  TGA
           stop       asp   stop              cys   stop                           stop

2041  GAT   /
      GAT  AAG  CTT   CGA   TTG   AAT   AGA   TTT   ATG   TTC   TTT   AGG   TAC
2071  GTA   TTC
```

FIG.2.

FIG.3.

FIG.4.

200K—

97.5K—
69K—

46K—

30K—

21K—

14K—

pKSD1014

pIN

pJY4306

pJY4310

pJY4285

pBC100

pBC207


SEQUENCE LISTINGS

(1) INFORMATION FOR SEQ ID NO:1:

    (i) SEQUENCE CHARACTERISTICS:
        (A) LENGTH: 2085 base pairs
        (B) TYPE: nucleic acid
        (C) STRANDEDNESS: double
        (D) TOPOLOGY: linear

    (ii) MOLECULE TYPE: protein

    (iii) HYPOTHETICAL: NO

    (iv) ANTI-SENSE: NO

    (vi) ORIGINAL SOURCE:
        (A) ORGANISM: Streptococcus pneumoniae
        (B) STRAIN: Rx1

    (vii) IMMEDIATE SOURCE:
        (B) CLONE: JY4313

    (ix) FEATURE:
        (A) NAME/KEY: intron
        (B) LOCATION: 1..2085

    (ix) FEATURE:
        (A) NAME/KEY: CDS
        (B) LOCATION: join(127..1984)

    (xi) SEQUENCE DESCRIPTION: SEQ ID NO:1:

```
AAGCTTATGA TATAGAAATT TGTAACAAAA ATGTAATATA AAACACTTGA CAAATATTTA      60

CGGAGGAGGC TTATACTTAA TATAAGTATA GTCTGAAAAT GACTATCAGA AAAGAGGTAA     120

ATTTAG ATG AAT AAG AAA AAA ATG ATT TTA ACA AGT CTA GCC AGC GTC       168
       Met Asn Lys Lys Lys Met Ile Leu Thr Ser Leu Ala Ser Val
        1               5                  10

CCT ATC TTA GGG GCT GGT TTT GTT GCG TCT CAG CCT ACT GTT GTA AGA     216
Ala Ile Leu Gly Ala Gly Phe Val Ala Ser Gln Pro Thr Val Val Arg
 15              20              25                  30

GCA GAA GAA TCT CCC GTA GCC AGT CAG TCT AAA GCT GAG AAA GAC TAT     264
Ala Glu Glu Ser Pro Val Ala Ser Gln Ser Lys Ala Glu Lys Asp Tyr
                35              40                  45

GAT GCA GCG AAG AAA GAT GCT AAG AAT GCG AAA AAA GCA GTA GAA GAT     312
Asp Ala Ala Lys Lys Asp Ala Lys Asn Ala Lys Lys Ala Val Glu Asp
            50              55                  60

GCT CAA AAG GCT TTA GAT GAT GCA AAA GCT GCT CAG AAA AAA TAT GAC     360
Ala Gln Lys Ala Leu Asp Asp Ala Lys Ala Ala Gln Lys Lys Tyr Asp
            65              70                  75

GAG GAT CAG AAG AAA ACT GAG GAG AAA GCC GCG CTA GAA AAA GCA GCG     408
Glu Asp Gln Lys Lys Thr Glu Glu Lys Ala Ala Leu Glu Lys Ala Ala
     80              85                  90
```

```
TCT GAA GAG ATG GAT AAG GCA GTG GCA GCA GTT CAA CAA GCG TAT CTA          456
Ser Glu Glu Met Asp Lys Ala Val Ala Ala Val Gln Gln Ala Tyr Leu
 95                 100                 105                 110

GCC TAT CAA CAA GCT ACA GAC AAA GCC GCA AAA GAC GCA GCA GAT AAG          504
Ala Tyr Gln Gln Ala Thr Asp Lys Ala Ala Lys Asp Ala Ala Asp Lys
                115                 120                 125

ATG ATA GAT GAA GCT AAG AAA CGC GAA GAA GAG GCA AAA ACT AAA TTT          552
Met Ile Asp Glu Ala Lys Lys Arg Glu Glu Glu Ala Lys Thr Lys Phe
            130                 135                 140

AAT ACT GTT CGA GCA ATG GTA GTT CCT GAG CCA GAG CAG TTG GCT GAG          600
Asn Thr Val Arg Ala Met Val Val Pro Glu Pro Glu Gln Leu Ala Glu
            145                 150                 155

ACT AAG AAA AAA TCA GAA GAA GCT AAA CAA AAA GCA CCA GAA CTT ACT          648
Thr Lys Lys Lys Ser Glu Glu Ala Lys Gln Lys Ala Pro Glu Leu Thr
    160                 165                 170

AAA AAA CTA GAA GAA GCT AAA GCA AAA TTA GAA GAG GCT GAG AAA AAA          696
Lys Lys Leu Glu Glu Ala Lys Ala Lys Leu Glu Glu Ala Glu Lys Lys
175                 180                 185                 190

GCT ACT GAA GCC AAA CAA AAA GTG GAT GCT GAA GAA GTC GCT CCT CAA          744
Ala Thr Glu Ala Lys Gln Lys Val Asp Ala Glu Glu Val Ala Pro Gln
                195                 200                 205

GCT AAA ATC GCT GAA TTG GAA AAT CAA GTT CAT AGA CTA GAA CAA GAG          792
Ala Lys Ile Ala Glu Leu Glu Asn Gln Val His Arg Leu Glu Gln Glu
            210                 215                 220

CTC AAA GAG ATT GAT GAG TCT GAA TCA GAA GAT TAT GCT AAA GAA GGT          840
Leu Lys Glu Ile Asp Glu Ser Glu Ser Glu Asp Tyr Ala Lys Glu Gly
            225                 230                 235

TTC CGT GCT CCT CTT CAA TCT AAA TTG GAT GCC AAA AAA GCT AAA CTA          888
Phe Arg Ala Pro Leu Gln Ser Lys Leu Asp Ala Lys Lys Ala Lys Leu
    240                 245                 250

TCA AAA CTT GAA GAG TTA AGT GAT AAG ATT GAT GAG TTA GAC GCT GAA          936
Ser Lys Leu Glu Glu Leu Ser Asp Lys Ile Asp Glu Leu Asp Ala Glu
255                 260                 265                 270

ATT GCA AAA CTT GAA GAT CAA CTT AAA GCT GCT GAA GAA AAC AAT AAT          984
Ile Ala Lys Leu Glu Asp Gln Leu Lys Ala Ala Glu Glu Asn Asn Asn
                275                 280                 285

GTA GAA GAC TAC TTT AAA GAA GGT TTA GAG AAA ACT ATT GCT GCT AAA         1032
Val Glu Asp Tyr Phe Lys Glu Gly Leu Glu Lys Thr Ile Ala Ala Lys
                290                 295                 300

AAA GCT GAA TTA GAA AAA ACT GAA GCT GAC CTT AAG AAA GCA GTT AAT         1080
Lys Ala Glu Leu Glu Lys Thr Glu Ala Asp Leu Lys Lys Ala Val Asn
            305                 310                 315

GAG CCA GAA AAA CCA GCT CCA GCT CCA GAA ACT CCA GCC CCA GAA GCA         1128
Glu Pro Glu Lys Pro Ala Pro Ala Pro Glu Thr Pro Ala Pro Glu Ala
            320                 325                 330
```

```
CCA GCT GAA CAA CCA AAA CCA GCG CCG GCT CCT CAA CCA GCT CCC GCA          1176
Pro Ala Glu Gln Pro Lys Pro Ala Pro Ala Pro Gln Pro Ala Pro Ala
335             340                 345                 350

CCA AAA CCA GAG AAG CCA GCT GAA CAA CCA AAA CCA GAA AAA ACA GAT          1224
Pro Lys Pro Glu Lys Pro Ala Glu Gln Pro Lys Pro Glu Lys Thr Asp
            355                 360                 365

GAT CAA CAA GCT GAA GAA GAC TAT GCT CGT AGA TCA GAA GAA GAA TAT          1272
Asp Gln Gln Ala Glu Glu Asp Tyr Ala Arg Arg Ser Glu Glu Glu Tyr
        370                 375                 380

AAT CGC TTG ACT CAA CAG CAA CCG CCA AAA GCT GAA AAA CCA GCT CCT          1320
Asn Arg Leu Thr Gln Gln Gln Pro Pro Lys Ala Glu Lys Pro Ala Pro
        385                 390                 395

GCA CCA AAA ACA GGC TGG AAA CAA GAA AAC GGT ATG TGG TAC TTC TAC          1368
Ala Pro Lys Thr Gly Trp Lys Gln Glu Asn Gly Met Trp Tyr Phe Tyr
        400                 405                 410

AAT ACT GAT GGT TCA ATG GCG ACA GGA TGG CTC CAA AAC AAC GGT TCA          1416
Asn Thr Asp Gly Ser Met Ala Thr Gly Trp Leu Gln Asn Asn Gly Ser
415                 420                 425                 430

TGG TAC TAC CTC AAC AGC AAT GGT GCT ATG GCT ACA GGT TGG CTC CAA          1464
Trp Tyr Tyr Leu Asn Ser Asn Gly Ala Met Ala Thr Gly Trp Leu Gln
                435                 440                 445

TAC AAT GGT TCA TGG TAT TAC CTC AAC GCT AAC GGC GCT ATG GCA ACA          1512
Tyr Asn Gly Ser Trp Tyr Tyr Leu Asn Ala Asn Gly Ala Met Ala Thr
        450                 455                 460

GGT TGG GCT AAA GTC AAC GGT TCA TGG TAC TAC CTC AAC GCT AAT GGT          1560
Gly Trp Ala Lys Val Asn Gly Ser Trp Tyr Tyr Leu Asn Ala Asn Gly
        465                 470                 475

GCT ATG GCT ACA GGT TGG CTC CAA TAC AAC GGT TCA TGG TAT TAC CTC          1608
Ala Met Ala Thr Gly Trp Leu Gln Tyr Asn Gly Ser Trp Tyr Tyr Leu
        480                 485                 490

AAC GCT AAC GGC GCT ATG GCA ACA GGT TGG GCT AAA GTC AAC GGT TCA          1656
Asn Ala Asn Gly Ala Met Ala Thr Gly Trp Ala Lys Val Asn Gly Ser
495                 500                 505                 510

TGG TAC TAC CTC AAC GCT AAT GGT GCT ATG GCT ACA GGT TGG CTC CAA          1704
Trp Tyr Tyr Leu Asn Ala Asn Gly Ala Met Ala Thr Gly Trp Leu Gln
                515                 520                 525

TAC AAC GGT TCA TGG TAC TAC CTC AAC GCT AAC GGT GCT ATG GCT ACA          1752
Tyr Asn Gly Ser Trp Tyr Tyr Leu Asn Ala Asn Gly Ala Met Ala Thr
        530                 535                 540

GGT TGG GCT AAA GTC AAC GGT TCA TGG TAC TAC CTC AAC GCT AAT GGT          1800
Gly Trp Ala Lys Val Asn Gly Ser Trp Tyr Tyr Leu Asn Ala Asn Gly
        545                 550                 555

GCT ATG GCA ACA GGT TGG GTG AAA GAT GGA GAT ACC TGG TAC TAT CTT          1848
Ala Met Ala Thr Gly Trp Val Lys Asp Gly Asp Thr Trp Tyr Tyr Leu
        560                 565                 570
```

```
GAA GCA TCA GGT GCT ATG AAA GCA AGC CAA TGG TTC AAA GTA TCA GAT        1896
Glu Ala Ser Gly Ala Met Lys Ala Ser Gln Trp Phe Lys Val Ser Asp
575             580             585             590

AAA TGG TAC TAT GTC AAT GGT TTA GGT GCC CTT GCA GTC AAC ACA ACT        1944
Lys Trp Tyr Tyr Val Asn Gly Leu Gly Ala Leu Ala Val Asn Thr Thr
            595             600             605

GTA GAT GGC TAT AAA GTC AAT GCC AAT GGT GAA TGG GTT TAA GCC GAT        1992
Val Asp Gly Tyr Lys Val Asn Ala Asn Gly Glu Trp Val *
            610             615

TAA ATT AAA GCA TGT TAA GAA CAT TTG ACA TTT TAA TTT TGA AAC AAA        2040

GAT AAG GTT CGA TTG AAT AGA TTT ATG TTC GTA TTC TTT AGG TAC           2085
```

(2) INFORMATION FOR SEQ ID NO:2:

    (i) SEQUENCE CHARACTERISTICS:
        (A) LENGTH: 619 amino acids
        (B) TYPE: amino acid
        (D) TOPOLOGY: linear

    (ii) MOLECULE TYPE: protein

    (xi) SEQUENCE DESCRIPTION: SEQ ID NO:2:

```
Met Asn Lys Lys Lys Met Ile Leu Thr Ser Leu Ala Ser Val Ala Ile
 1           5               10              15

Leu Gly Ala Gly Phe Val Ala Ser Gln Pro Thr Val Val Arg Ala Glu
            20              25              30

Glu Ser Pro Val Ala Ser Gln Ser Lys Ala Glu Lys Asp Tyr Asp Ala
        35              40              45

Ala Lys Lys Asp Ala Lys Asn Ala Lys Lys Ala Val Glu Asp Ala Gln
    50              55              60

Lys Ala Leu Asp Asp Ala Lys Ala Ala Gln Lys Lys Tyr Asp Glu Asp
65              70              75              80

Gln Lys Lys Thr Glu Glu Lys Ala Ala Leu Glu Lys Ala Ala Ser Glu
            85              90              95

Glu Met Asp Lys Ala Val Ala Ala Val Gln Gln Ala Tyr Leu Ala Tyr
            100             105             110

Gln Gln Ala Thr Asp Lys Ala Ala Lys Asp Ala Ala Asp Lys Met Ile
        115             120             125

Asp Glu Ala Lys Lys Arg Glu Glu Glu Ala Lys Thr Lys Phe Asn Thr
        130             135             140

Val Arg Ala Met Val Val Pro Glu Pro Glu Gln Leu Ala Glu Thr Lys
145             150             155             160
```

16

```
Lys Lys Ser Glu Glu Ala Lys Gln Lys Ala Pro Glu Leu Thr Lys Lys
                165             170                 175

Leu Glu Glu Ala Lys Ala Lys Leu Glu Glu Ala Glu Lys Lys Ala Thr
            180             185                 190

Glu Ala Lys Gln Lys Val Asp Ala Glu Glu Val Ala Pro Gln Ala Lys
        195             200                 205

Ile Ala Glu Leu Glu Asn Gln Val His Arg Leu Glu Gln Glu Leu Lys
    210             215                 220

Glu Ile Asp Glu Ser Glu Ser Glu Asp Tyr Ala Lys Glu Gly Phe Arg
225             230             235                 240

Ala Pro Leu Gln Ser Lys Leu Asp Ala Lys Lys Ala Lys Leu Ser Lys
            245             250                 255

Leu Glu Glu Leu Ser Asp Lys Ile Asp Glu Leu Asp Ala Glu Ile Ala
            260             265                 270

Lys Leu Glu Asp Gln Leu Lys Ala Ala Glu Glu Asn Asn Asn Val Glu
        275             280                 285

Asp Tyr Phe Lys Glu Gly Leu Glu Lys Thr Ile Ala Ala Lys Lys Ala
    290             295             300

Glu Leu Glu Lys Thr Glu Ala Asp Leu Lys Lys Ala Val Asn Glu Pro
305             310                 315                 320

Glu Lys Pro Ala Pro Ala Pro Glu Thr Pro Ala Pro Glu Ala Pro Ala
            325             330                 335

Glu Gln Pro Lys Pro Ala Pro Ala Pro Gln Pro Ala Pro Ala Pro Lys
            340         -         - 345                 350

Pro Glu Lys Pro Ala Glu Gln Pro Lys Pro Glu Lys Thr Asp Asp Gln
        355             360             365

Gln Ala Glu Glu Asp Tyr Ala Arg Arg Ser Glu Glu Glu Tyr Asn Arg
    370             375             380

Leu Thr Gln Gln Gln Pro Pro Lys Ala Glu Lys Pro Ala Pro Ala Pro
385             390             395                 400

Lys Thr Gly Trp Lys Gln Glu Asn Gly Met Trp Tyr Phe Tyr Asn Thr
            405             410                 415

Asp Gly Ser Met Ala Thr Gly Trp Leu Gln Asn Asn Gly Ser Trp Tyr
            420             425             430

Tyr Leu Asn Ser Asn Gly Ala Met Ala Thr Gly Trp Leu Gln Tyr Asn
        435             440             445

Gly Ser Trp Tyr Tyr Leu Asn Ala Asn Gly Ala Met Ala Thr Gly Trp
    450             455             460

Ala Lys Val Asn Gly Ser Trp Tyr Tyr Leu Asn Ala Asn Gly Ala Met
465             470             475                 480
```

Ala Thr Gly Trp Leu Gln Tyr Asn Gly Ser Trp Tyr Tyr Leu Asn Ala
                485             490                         495

Asn Gly Ala Met Ala Thr Gly Trp Ala Lys Val Asn Gly Ser Trp Tyr
            500             505                 510

Tyr Leu Asn Ala Asn Gly Ala Met Ala Thr Gly Trp Leu Gln Tyr Asn
        515             520             525

Gly Ser Trp Tyr Tyr Leu Asn Ala Asn Gly Ala Met Ala Thr Gly Trp
    530             535                 540

Ala Lys Val Asn Gly Ser Trp Tyr Tyr Leu Asn Ala Asn Gly Ala Met
545             550             555                     560

Ala Thr Gly Trp Val Lys Asp Gly Asp Thr Trp Tyr Tyr Leu Glu Ala
            565             570             575

Ser Gly Ala Met Lys Ala Ser Gln Trp Phe Lys Val Ser Asp Lys Trp
            580             585             590

Tyr Tyr Val Asn Gly Leu Gly Ala Leu Ala Val Asn Thr Thr Val Asp
        595             600             605

Gly Tyr Lys Val Asn Ala Asn Gly Glu Trp Val
    610             615


(3) INFORMATION FOR SEQ ID NO:3:

        (i) SEQUENCE CHARACTERISTICS:
            (A) LENGTH: 33 base pairs
            (B) TYPE: nucleic acid
            (C) STRANDEDNESS: single
            (D) TOPOLOGY: linear

        (ii) MOLECULE TYPE: DNA (genomic)


        (xi) SEQUENCE DESCRIPTION: SEQ ID NO:3:

GCGCGTCGAC GGCTTAAACC CATTCACCAT TGG                                          33

(4) INFORMATION FOR SEQ ID NO:4:

        (i) SEQUENCE CHARACTERISTICS:
            (A) LENGTH: 28 base pairs
            (B) TYPE: nucleic acid
            (C) STRANDEDNESS: single
            (D) TOPOLOGY: linear

        (ii) MOLECULE TYPE: DNA (genomic)


        (xi) SEQUENCE DESCRIPTION: SEQ ID NO:4:

CCGGATCCTG AGCCAGAGCA GTTGGCTG                                                28

(5) INFORMATION FOR SEQ ID NO:5:

    (i) SEQUENCE CHARACTERISTICS:
        (A) LENGTH: 31 base pairs
        (B) TYPE: nucleic acid
        (C) STRANDEDNESS: single
        (D) TOPOLOGY: linear

    (ii) MOLECULE TYPE: DNA (genomic)


    (xi) SEQUENCE DESCRIPTION: SEQ ID NO:5:

CCGGATCCGC TCAAAGAGAT TGATGAGTCT G

                                                                31